# EUROPEAN PATENT APPLICATION

(11) **EP 3 267 200 A1**
(43) Date of publication of application: **10.01.2018**
(21) Application number: 16761673.9
(22) Date of filing: 04.03.2016
(51) Int. Cl.: G01N 33/92, G01N 33/50

(54) **BIOMARKER OF DEMENTIA WITH LEWY BODIES**

(30) Priority: 06.03.2015 JP 2015044353
(71) Applicant: Niigata University, Niigata-shi Niigata 950-2181 (JP); Eisai R&D Management Co., Ltd., Tokyo 112-8088 (JP)
(72) Inventor: IKEUCHI Takeshi, Niigata-shi Niigata 951-8585 (JP); KUWANO Ryozo, Niigata-shi Niigata 951-8585 (JP); SATO Yoshiaki, Tsukuba-shi Ibaraki 300-2635 (JP); BERNIER Francois, Tsukuba-shi Ibaraki 300-2635 (JP)
(74) Representative: Hoffmann Eitle
(86) International application number: PCT/JP2016/056828
(87) International publication number: WO 2016/143703

(57) **Abstract**

Cholesterol and desmosterol levels, and the ratio of these levels can be used as a blood biomarker for Alzheimer's disease, frontotemporal lobar degeneration, or dementia with Lewy bodies.

## Description

### Technical Field

The present invention relates to a biomarker useful for diagnosis of dementia with Lewy bodies (DLB). More specifically, the present invention relates to a method for evaluating an effect of a candidate substance of a therapeutic agent for DLB, a method for assisting diagnosis of DLB, a method for diagnosing DLB, a method for selecting a patient that is likely to benefit from a therapeutic effect of a candidate substance of a therapeutic agent for DLB, and a method for distinguishing DLB from Alzheimer's disease (AD) and frontotemporal lobar degeneration (FTLD).

### Background Art

Biomarkers are very useful for diagnosing diseases and determining the state of a disease and are important in selecting patients to be treated, assisting selection of a suitable treatment, monitoring side effects, and discovering new drugs.

Pathological features of dementia with Lewy bodies include wide appearance of a great number of Lewy bodies in central nervous system, including cerebral cortex, amygdala, and nucleus basalis of Meynert, and in autonomic nervous system. Dementia with Lewy bodies has symptoms such as progressive decrease in cognitive function, marked fluctuation of attention or arousal level, delusion based on specific visual hallucination, idiopathic parkinsonian syndromes (such as hand tremor and gait disturbance), REM sleep behavior disorder, hypersensitivity to antipsychotics, autonomic symptoms, and depression. It has been reported that patients with dementia with Lewy bodies have significant increase in oxidized α-helical Aβ1-40 in cerebrospinal fluid and decrease in α-synuclein in blood as compared with elderly healthy subjects (Non Patent Literature 1 and 2). It has been also reported that coenzyme Q10 (CoQ10) in serum from patients with dementia with Lewy bodies is statistically significantly decreased as compared with elderly healthy subjects but the ratio of CoQ10 to cholesterol (CHO) (CoQ10/CHO in patients with dementia with Lewy bodies is not statistically different from the ratio in elderly healthy subjects and furthermore no correlation has been found between CoQ10 or CoQ10/CHO and age at onset, Mini Mental State Examination (MMSE) score, or the like. CHO is not mentioned in the experimental results of the report as described above, and a slight decrease in CHO levels in patients with dementia with Lewy bodies as compared with elderly healthy subjects is mentioned in the discussion of the report but is not mentioned to be statistically significant (Non Patent Literature 3).

Alzheimer's disease is a neurodegenerative disease characterized by slow development of disease characterized by impairment in recent memory from early stage and continuous decrease in cognitive function. Amyloid beta (Aβ) 1-42 and phosphorylated tau protein in cerebrospinal fluid are believed to be useful biomarkers for Alzheimer's disease (Non Patent Literature 4 and 5). It is also believed that abnormality of cholesterol metabolism occurs in Alzheimer's disease. It has been reported that patients with Alzheimer's disease have unchanged desmosterol (DES) levels in blood plasma and decreased desmosterol levels in cerebrospinal fluid as compared with elderly healthy subjects (Non Patent Literature 6). On the other hand, it has been also reported that desmosterol levels in blood plasma from patients with Alzheimer's disease are lower than those in elderly healthy subjects (Non Patent Literature 7 and Patent Literature 1).

Frontotemporal lobar degeneration is a disease that exhibits progressive degeneration localized to frontal lobe and temporal lobe and includes degenerative dementia of non-Alzheimer's type whose cardinal sign comprises clinical behavior disorder and language disorder. Tau protein and TAR DNA binding protein of 43kD (TDP-43) have been identified as a biomarker in cerebrospinal fluid (Non Patent Literature 8). It has further been indicated that phosphorylated TDP-43 in blood plasma will significantly correlate with the severity score of phosphorylated TDP-43 pathology in brain and may be useful as a severity biomarker (Non Patent Literature 9 and 10). However, specificity of the above-mentioned biomarkers in distinguishing among dementia with Lewy bodies, Alzheimer's disease, and frontotemporal lobar degeneration has not been revealed yet.

### Citation List

### Patent Literature

Patent Literature 1: U.S. Patent Application Publication No. 2013/0266589

### Non Patent Literature

Non Patent Literature 1: Bibl, M. et al., "CSF amyloid-beta-peptides in Alzheimer's disease, dementia with Lewy bodies and Parkinson's disease dementia" Brain, (2006) 129, 1177-1187
Non Patent Literature 2: Laske, C. et al., "Decreased alpha-synuclein serum levels in patients with Lewy body dementia compared to Alzheimer's disease patients and control subjects. Dement. Geriatr. Cogn. Disord. (2011) 31, 413-416.
Non Patent Literature 3: Molina, J. A. et al., "Serum levels of coenzyme Q in patients with Lewy body disease. J. Neural Transm. (2002) 109, 1195-1201.
Non Patent Literature 4: Ibach, B. et al., "Cerebrospinal fluid tau and beta-amyloid in Alzheimer patients, disease controls and an age-matched random sample" Neurobiol. Aging (2006), 27(9), 1202-1211
Non Patent Literature 5: Bouwman, F. H. et al., "CSF biomarker levels in early and late onset Alzheimer's disease", Neurobiol. Aging (2009), 30(12), 1895-1901
Non Patent Literature 6: Koelsch, H. et al., "Alterations of cholesterol precursor levels in Alzheimer's disease", Biochim. Biophys. Acta (2010), 1801(8), 945-950
Non Patent Literature 7: Sato, Y. et al., "Identification of a new plasma biomarker of Alzheimer's disease using metabolomics technology", J. Lipid Res. (2012), 53(3), 567-576
Non Patent Literature 8: Hu, W. T. et al., "Novel CSF biomarkers for frontotemporal lobar degenerations", Neurology (2010), 75, 2079-2086
Non Patent Literature 9: Foulds, P. et al., "TDP-43 protein in plasma may index TDP-43 brain pathology in Alzheimer's disease and frontotemporal lobar degeneration", Acta Neuropathol. (2008), 116, 141-146
Non Patent Literature 10: Foulds, P. et al., "Plasma phosphorylated-TDP-43 protein levels correlate with brain pathology in frontotemporal lobar degeneration", Acta Neuropathol. (2009), 118, 647-658

### Summary of Invention

### Technical Problem

Biomarkers useful for diagnosis of dementia with Lewy bodies and biomarkers useful for distinguishing among Alzheimer's disease, frontotemporal lobar degeneration, and dementia with Lewy bodies are demanded. Furthermore, less invasive biomarkers using blood or the like are demanded because collection of cerebrospinal fluid from patients for measuring biomarkers will damage the patients.

### Solution to Problem

The present inventors have demonstrated that the cholesterol levels and desmosterol levels in blood from patients with dementia with Lewy bodies are respectively statistically significantly lower than the cholesterol levels and desmosterol levels in blood from elderly healthy subjects, and have found that the cholesterol level and desmosterol level in blood can be used as a biomarker for patients with dementia with Lewy bodies.

The present inventors have found that patients with dementia with Lewy bodies and patients with Alzheimer's disease or patients with frontotemporal lobar degeneration can be distinguished based on the finding that cholesterol levels in blood from patients with dementia with Lewy bodies are statistically significantly lower than those levels in elderly healthy subjects while cholesterol levels in blood from patients with Alzheimer's disease and patients with frontotemporal lobar degeneration are not respectively statistically different from those levels in elderly healthy subjects.

The present inventors have found that desmosterol levels can be utilized for distinguishing between a patient with dementia with Lewy bodies and a patient with frontotemporal lobar degeneration, and between a patient with Alzheimer's disease and a patient with frontotemporal lobar degeneration based on the finding that desmosterol levels in blood from patients with dementia with Lewy bodies and patients with Alzheimer's disease are statistically significantly lower than those levels in elderly healthy subjects while desmosterol levels in blood from patients with frontotemporal lobar degeneration are not statistically different from those levels in elderly healthy subjects.

The present inventors have found that a ratio of a desmosterol level to a cholesterol level can be utilized for distinguishing between a patient with Alzheimer's disease and a patient with dementia with Lewy bodies and between a patient with Alzheimer's disease and a patient with frontotemporal lobar degeneration based on the finding that the ratios of desmosterol levels to cholesterol levels in blood from patients with Alzheimer's disease are statistically significantly lower than those ratios in elderly healthy subjects while the ratios of desmosterol levels to cholesterol levels in blood from patients with dementia with Lewy bodies and patients with frontotemporal lobar degeneration are not statistically different from those ratios in elderly healthy subjects.

In other words, the present invention provides the following [1] to [8].
[1] A method for evaluating an effect of a candidate substance of a therapeutic agent for dementia with Lewy bodies, comprising:
   measuring a desmosterol (DES) level and cholesterol (CHO) level in blood from a non-human animal before administration of the candidate substance;
   measuring the DES level and CHO level in blood from the non-human animal after administration of the candidate substance; and
   providing an indication that the candidate substance is likely to be effective in treatment of dementia with Lewy bodies when at least one selected from the DES level and CHO level in blood from the non-human animal after administration of the candidate substance is increased as compared with the DES level and CHO level in blood from the non-human animal before administration of the candidate substance.
[2] A method for evaluating an effect of a candidate substance of a therapeutic agent for dementia with Lewy bodies, comprising:
   measuring a DES level and CHO level in blood from a first non-human animal to which the candidate substance is not administered;
   measuring the DES level and CHO level in blood from a second non-human animal to which the candidate substance is not administered; and
   providing an indication that the candidate substance is likely to be effective in treatment of dementia with Lewy bodies when at least one selected from the DES level and CHO level in blood from the second non-human animal to which the candidate substance is administered is higher than the DES level and CHO level in blood from the first non-human animal to which the candidate substance is not administered.
[3] A method for evaluating an effect of a candidate substance of a therapeutic agent for dementia with Lewy bodies, comprising:
   measuring a DES level and CHO level in blood from a subject before administration of the candidate substance;
   measuring the DES level and CHO level in blood from the subject after administration of the candidate substance;
   providing an indication that the candidate substance is likely to be effective in treatment of dementia with Lewy bodies when at least one selected from the DES level and CHO level in blood from the subject after administration of the candidate substance is increased as compared with the DES level and CHO level in blood from the subject before administration of the candidate substance.
[4] A method for assisting diagnosis of dementia with Lewy bodies, comprising:
   measuring a DES level and CHO level in blood from a subject and
   providing an indication that the subject is likely to have dementia with Lewy bodies when at least one selected from the DES level and CHO level in blood from the subject is lower than a reference value.
[5] A method for diagnosing dementia with Lewy bodies, comprising:
   measuring a DES level and CHO level in blood from a subject and
   determining that the subject has dementia with Lewy bodies when at least one selected from the DES level and CHO level in blood from the subject is lower than a reference value.
[6] A method for selecting a patient that is likely to benefit from a therapeutic effect of a candidate substance of a therapeutic agent for dementia with Lewy bodies, comprising:
   measuring a DES level and CHO level in blood from a subject and
   providing an indication that the subject is a patient to be administered with the candidate substance of a therapeutic agent for dementia with Lewy bodies when at least one selected from the DES level and CHO level in blood from the subject is lower than a reference value.
[7] A method for distinguishing among dementia with Lewy bodies, Alzheimer's disease, and frontotemporal lobar degeneration, comprising:
   measuring a DES level and CHO level in blood from a subject and
   providing an indication
      (1) that the subject is likely to have dementia with Lewy bodies rather than the Alzheimer's disease and the frontotemporal lobar degeneration when the CHO level in blood from the subject is lower than a reference value; or
      (2) that the subject is likely to have dementia with Lewy bodies rather than the Alzheimer's disease and the frontotemporal lobar degeneration when the DES level in blood from the subject is lower than a reference value and a ratio of the DES level to the CHO level (DES/CHO) is not different from a reference ratio.
[8] A method for distinguishing among dementia with Lewy bodies, Alzheimer's disease, and frontotemporal lobar degeneration, comprising:
   measuring a DES level and CHO level in blood from a subject and
   providing an indication
      (1) that the subject is likely to have dementia with Lewy bodies rather than Alzheimer's disease and frontotemporal lobar degeneration when the CHO level in blood from the subject is lower than a reference value;
      (2) that the subject is likely to have dementia with Lewy bodies or Alzheimer's disease rather than frontotemporal lobar degeneration when the DES level in blood from the subject is lower than a reference value;
      (3) that the subject is likely to have dementia with Lewy bodies rather than Alzheimer's disease and frontotemporal lobar degeneration when the DES level in blood from the subject is lower than a reference value and DES/CHO is not different from a reference value; or
      (4) that the subject is likely to have Alzheimer's disease rather than dementia with Lewy bodies and frontotemporal lobar degeneration when DES/CHO in blood from the subject is lower than a reference value.

### Advantageous Effects of Invention

The cholesterol level and desmosterol level in blood may be biomarkers for dementia with Lewy bodies. More specifically, the biomarkers as described above can assist diagnosis of dementia with Lewy bodies, can diagnose dementia with Lewy bodies, can select patients that is likely to benefit from a therapeutic effect of a candidate substance of a therapeutic agent for dementia with Lewy bodies, and can evaluate an effect of candidate substances of therapeutic agents for dementia with Lewy bodies.

Moreover, the biomarkers can distinguish among a patient with dementia with Lewy bodies, a patient with frontotemporal lobar degeneration, and a patient with Alzheimer's disease.

The biomarkers use cholesterol levels or desmosterol levels in blood, and they therefore require no collection of cerebrospinal fluid and are less invasive.

### Brief Description of Drawings

[Figure 1] Figure 1 shows a receiver operating characteristic curve of cholesterol levels in blood plasma from elderly healthy subjects and patients with dementia with Lewy bodies.
[Figure 2] Figure 2 shows a receiver operating characteristic curve of cholesterol levels in blood plasma from patients with Alzheimer's disease and patients with dementia with Lewy bodies.
[Figure 3] Figure 3 shows a receiver operating characteristic curve of cholesterol levels in blood plasma from patients with frontotemporal lobar degeneration and patients with dementia with Lewy bodies.

### Description of Embodiments

The method for evaluating an effect of a candidate substance of a therapeutic agent for dementia with Lewy bodies according to an embodiment of the present invention is performed on non-human animals or subjects. It is preferable that the non-human animals are mammals and it is more preferable that the non-human animals are model animals of dementia with Lewy bodies. It is preferable that the subjects are human patients suspected to have dementia with Lewy bodies.

The model animals of dementia with Lewy bodies that can be utilized include model animals of Alzheimer's disease and model animals of Parkinson's disease. Specifically, examples of the model animals of Alzheimer's disease include animals that have increased production of amyloid beta due to modification of amyloid precursor protein (APP), presenilin 2, and the like and Tg2576, a genetically modified mouse over-expressing APP. Furthermore, examples of the model animals of Parkinson's disease include an α-synuclein transgenic mouse over-expressing α-synuclein.

In the method for evaluating an effect of a candidate substance of a therapeutic agent for dementia with Lewy bodies according to an embodiment of the present invention, first, using non-human animals or subject, the desmosterol level and cholesterol level are measured in blood from an identical individual before and after administration of the candidate substance. An indication that the candidate substance is likely to be effective in treatment of dementia with Lewy bodies is provided when at least one of the desmosterol level and cholesterol level in blood after administration of the candidate substance is higher than the level before administration of the candidate substance. The effect of the candidate substance of the therapeutic agent for dementia with Lewy bodies can be evaluated based on the provided indication.

In another embodiment of the method for evaluating an effect of a candidate substance of a therapeutic agent for dementia with Lewy bodies, first, using non-human animals or subject of different individuals, desmosterol levels and cholesterol levels in blood from one or more individuals to which the candidate substance is not administered (the candidate substance non-administered group) are measured, and desmosterol levels and cholesterol levels in blood from one or more other individuals to which the candidate substance is administered (the candidate substance-administered group) after administration of the candidate substance are measured. The desmosterol levels and cholesterol levels in the candidate substance non-administered group and the candidate substance-administered group are compared. An indication that the candidate substance is likely to be effective in treatment of dementia with Lewy bodies is provided when at least one of the desmosterol level and cholesterol level in blood from the candidate substance-administered group is higher than the level in the candidate substance non-administered group. The effect of the candidate substance can be evaluated based on the provided indication.

The candidate substances of therapeutic agents for dementia with Lewy bodies according to embodiments of the present invention are not particularly limited but are a cholesterol metabolic pathway modulator or a modulator based on Aβ hypothesis, preferably a β-secretase inhibitor, a γ-secretase inhibitor, a γ-secretase modulator, an anti-Aβ antibody, an anti-Aβ oligomer antibody, or the like. The candidate substances are further levodopa, a dopamine agonist, a monoamine oxidase B inhibitor, or the like.

The method for assisting diagnosis of dementia with Lewy bodies according to an embodiment of the present invention is performed on subjects. It is preferable that the subjects are human patients suspected to have dementia with Lewy bodies. In the method, first, the desmosterol level and cholesterol level in blood from the subject are measured, and the levels are compared with their reference values to make identification. An indication that the subject is likely to have dementia with Lewy bodies is provided when at least one of the desmosterol level and cholesterol level in the subject is lower than their respective reference values. Diagnosis of dementia with Lewy bodies can be assisted based on the provided indication.

The method for diagnosing dementia with Lewy bodies according to an embodiment of the present invention is performed on subjects. It is preferable that the subjects are human patients suspected to have dementia with Lewy bodies. In the method, first, the desmosterol level and cholesterol level in blood from the subject are measured, and the levels are compared with their reference values to make identification. In other words, the subject is identified as having dementia with Lewy bodies when at least one of the desmosterol level and cholesterol level in the subject is lower than their respective reference values. The subject can be diagnosed as having dementia with Lewy bodies based on the determination.

The method for selecting patients that is likely to benefit from a therapeutic effect of a candidate substance of a therapeutic agent for dementia with Lewy bodies according to an embodiment of the present invention is performed on subjects. It is preferable that the subjects are human patients that have been diagnosed as dementia with Lewy bodies. In the method, first, the desmosterol level and cholesterol level in blood from the subjects are measured, and the levels are compared with their reference values to make identification. In other words, an indication that the subject is a patient to be administered with the candidate substance of the therapeutic agent for dementia with Lewy bodies is provided when at least one of the desmosterol level and cholesterol level in the subject is lower than their respective reference values. Patients to be administered the candidate substance of the therapeutic agent for dementia with Lewy bodies, namely patients that is likely to benefit from a therapeutic effect of the candidate substance can be selected based on the provided indication.

The method for distinguishing among dementia with Lewy bodies, Alzheimer's disease, and frontotemporal lobar degeneration according to an embodiment of the present invention is performed on subjects. It is preferable that the subjects are human patients suspected to have dementia with Lewy bodies, Alzheimer's disease, or frontotemporal lobar degeneration. In the method, first, the desmosterol level and cholesterol level in blood from the subject are measured, and the cholesterol level is compared with its reference value to make identification. In other words, an indication that the subject is likely to have dementia with Lewy bodies rather than Alzheimer's disease and frontotemporal lobar degeneration is provided when the cholesterol level in blood from the subject is lower than its reference value. Dementia with Lewy bodies, Alzheimer's disease, and frontotemporal lobar degeneration can be distinguished based on the provided indication.

In another embodiment, in the method for distinguishing among dementia with Lewy bodies, Alzheimer's disease, and frontotemporal lobar degeneration, first, the desmosterol level and cholesterol level in blood from a subject are measured, the desmosterol level and the ratio of desmosterol level to cholesterol level are compared with their respective reference values to make identification. In other words, an indication that the subject is likely to have dementia with Lewy bodies rather than Alzheimer's disease and frontotemporal lobar degeneration is provided when the desmosterol level in blood from the subject is lower than its reference value and the ratio of desmosterol level to cholesterol level is not different from its reference value. Dementia with Lewy bodies, Alzheimer's disease, and frontotemporal lobar degeneration can be distinguished based on the provided indication.

In yet another embodiment, in the method for distinguishing among dementia with Lewy bodies, Alzheimer's disease, and frontotemporal lobar degeneration, first, the desmosterol level and cholesterol level in blood from a subject are measured, and the levels are compared with their respective reference values to make identification. In other words, an indication that the subject is likely to have dementia with Lewy bodies or Alzheimer's disease rather than frontotemporal lobar degeneration is provided when the desmosterol level in blood from the subject is lower than its reference value. Dementia with Lewy bodies, Alzheimer's disease, and frontotemporal lobar degeneration can be distinguished based on the provided indication.

In further another embodiment, in the method for distinguishing among dementia with Lewy bodies, Alzheimer's disease, and frontotemporal lobar degeneration, first, the desmosterol level and cholesterol level in blood from a subject are measured, and the levels are compared with their respective reference values to make identification. In other words, an indication that the subject is likely to have Alzheimer's disease rather than dementia with Lewy bodies and frontotemporal lobar degeneration is provided when the desmosterol level in blood from the subject is lower than its reference value and the ratio of desmosterol level to cholesterol level is not different from its reference value. Dementia with Lewy bodies, Alzheimer's disease, and frontotemporal lobar degeneration can be distinguished based on the provided indication.

Two or more indications as described above may be combined in the method for distinguishing among dementia with Lewy bodies, Alzheimer's disease, and frontotemporal lobar degeneration according to an embodiment of the present invention. For example, an indication that the subject is likely to have dementia with Lewy bodies rather than Alzheimer's disease and frontotemporal lobar degeneration, wherein the indication is provided when the cholesterol level in blood from the subject is lower than its reference value, and an indication that the subject is likely to have dementia with Lewy bodies rather than Alzheimer's disease and frontotemporal lobar degeneration, wherein the indication is provided when the desmosterol level in blood from the subject is lower than its reference value and the ratio of desmosterol level to cholesterol level is not different from its reference value, can be combined. Furthermore, for example, in addition to these indications, an indication that the subject is likely to have dementia with Lewy bodies or Alzheimer's disease rather than frontotemporal lobar degeneration, wherein the indication is provided when the desmosterol level in blood from the subject is lower than its reference value, and an indication that the subject is likely to have Alzheimer's disease rather than dementia with Lewy bodies and frontotemporal lobar degeneration, wherein the indication is provided when the desmosterol level in blood from the subject is lower than its reference value and the ratio of desmosterol level to cholesterol level is not different from its reference value, can be further combined.

The cholesterol level and desmosterol level in blood according to an embodiment of the present invention can be measured by liquid chromatography/mass spectroscopy (LC/MS). More specifically, these levels can be measured under a condition described in the Examples.

The cholesterol level and desmosterol level in blood can be also measured by enzyme-linked immunosorbent assay (ELISA) or gas chromatography/mass spectroscopy (GC-MS).

The cholesterol level and desmosterol level in blood are levels in whole blood, levels in blood serum, or levels in blood plasma, are preferably levels in blood serum or levels in blood plasma, and are more preferably levels in blood plasma.

In the methods as described above, the cholesterol level and desmosterol level in blood may be used as a biomarker, and the ratio between the cholesterol level and the desmosterol level may be used as a biomarker.

The elderly healthy subjects are adult humans aged 65 years or older that have no symptoms of dementia with Lewy bodies, Alzheimer's disease, and frontotemporal lobar degeneration.

The reference values of biomarkers can be derived by referring the levels in elderly healthy subjects and the levels in patients that have been definitively diagnosed as dementia with Lewy bodies, Alzheimer's disease, or frontotemporal lobar degeneration.

The reference values of biomarkers can be derived by referring the values in elderly healthy subjects or patients that have been definitively diagnosed as dementia with Lewy bodies, Alzheimer's disease, or frontotemporal lobar degeneration. Specific examples of the reference values of biomarkers are not particularly limited but include mean, mean ± 1 standard deviation, mean ± 2 standard deviations, or mean ± 3 standard deviations of the biomarkers in elderly healthy subjects or patients with dementia with Lewy bodies, Alzheimer's disease, or frontotemporal lobar degeneration.

Furthermore, other specific examples of the reference values of biomarkers include a cut-off value calculated from a receiver operating characteristic curve (ROCC) of elderly healthy subjects and dementia with Lewy bodies; elderly healthy subjects and Alzheimer's disease; or elderly healthy subjects and frontotemporal lobar degeneration (Akobeng, A. K., Acta Paediatr. (2007) 96, 644-647).

The receiver operating characteristic curve will be depicted in a graph with sensitivity on the Y axis and 1-specificity on the X axis. As the sensitivity is maximized, the Y value of the receiver operating characteristic curve is maximized. As the specificity is maximized, the X value of the receiver operating characteristic curve is correspondingly minimized. Therefore, the value of the point on the Y axis closest to the left upper corner of the receiver operating characteristic curve will be suitable for the first candidate of the cut-off value. However, even when the cut-off value is used as a reference value, setting of the reference value is varied depending on decreasing false-negative detection of patients with dementia with Lewy bodies, Alzheimer's disease, or frontotemporal lobar degeneration or excluding patients that are less likely to have dementia with Lewy bodies, Alzheimer's disease, or frontotemporal lobar degeneration. For example, it is important that sensitivity rather than specificity is maximized (false-negative detection is minimized) in order to decrease false-negative detection of patients with dementia with Lewy bodies, Alzheimer's disease, or frontotemporal lobar degeneration. In this case, a suitable cut-off value on the receiver operating characteristic curve will transfer from the neighborhood of the left upper corner to the right upper corner. On the other hand, it is important that specificity is maximized (false-positive detection is minimized) in order to exclude patients that are less likely to have dementia with Lewy bodies, Alzheimer's disease, or frontotemporal lobar degeneration. In this case, a suitable cut-off value on the receiver operating characteristic curve will transfer to the left bottom corner.

As mentioned above, the reference values are not particularly limited but can be derived by referring the values in elderly healthy subjects and the values in patients that have been definitively diagnosed as Alzheimer's disease, frontotemporal lobar degeneration, or dementia with Lewy bodies.

### Examples

Example 1: Measurement of cholesterol levels and desmosterol levels in blood from elderly healthy subjects, patients with dementia with Lewy bodies, patients with Alzheimer's disease, or patients with frontotemporal lobar degeneration

### (1) Samples for measurement

Blood plasma from 30 elderly healthy subjects, 118 patients with Alzheimer's disease, 48 patients with frontotemporal lobar degeneration, and 12 patients with dementia with Lewy bodies were used for measurement.

### (2) Measurement of cholesterol levels and desmosterol levels

To 25 µL of blood plasma from elderly healthy subjects, patients with dementia with Lewy bodies, patients with Alzheimer's disease, or patients with frontotemporal lobar degeneration were added 100 µL of 200 µg/mL cholesterol-d7 (KANTO CHEMICAL CO., INC.) and 100 µL of 200 ng/mL desmosterol-d6 (Avanti Polar Lipids, Inc.) as internal standards, and further added 100 µL of 50% potassium hydroxide aqueous solution, and the mixture was incubated at 70°C for 60 minutes. Subsequently, the target substance was concentrated by liquid-liquid extraction or solid phase extraction, and liquid chromatography/atmospheric pressure chemical ionization mass spectroscopy (LC/APCI-MS) was performed. In liquid-liquid extraction, to the incubated samples (solutions), 2 mL of hexane and 0.5 mL of phosphate buffered physiological saline (pH 6.8) were added, and the mixture was stirred and centrifuged before removing the hexane layer. This extraction procedure was repeated twice. The resulting hexane layers were combined, and the solvent was evaporated to dryness with nitrogen gas before dissolving the pellet in ethanol. In solid phase extraction, the incubated sample (solution) was passed through a C18 solid phase extraction cartridge before washing it with 45% ethanol to elute the target substance with 100 µL of ethanol.

The apparatus used for LC/APCI-MS was LC-20AD system (manufactured by SHIMADZU CORPORATION) equipped with auto sampler SIL-20AC, column oven CTO-20AC, and quadrupole mass spectrometer LCMS-2010EV. The column used was YMC-Pack Pro C18 RS column (4.6 mm, I.D., 250 mm long, manufactured by YMC) or Kinetex 2.6 XB-C18 (3.0 mm, I.D., 150 mm long, manufactured by SHIMADZU CORPORATION), and the column temperature was set to 50°C or 40°C. A mixed solvent of water and methanol (flow rate: 1 mL/minute) was used as a mobile phase in the analysis on the YMC-Pack Pro C18 RS. More specifically, solvent A (water: methanol = 50:50) and solvent B (methanol) were used, and 85% B was used from 0 to 45 minutes, 100% B was used from 45 to 55 minutes, and 85% B was used from 55 to 70 minutes. A mixed solvent of water, acetonitrile, and methanol (flow rate: 0.9 mL/minute) was used as a mobile phase in the analysis on the Kinetex 2.6 XB-C18. More specifically, solvent A (water: acetonitrile = 50:50) and solvent B (methanol: acetonitrile = 50: 50) were used, and 70% B was used from 0 to 20 minutes, 100% B was used from 20.1 to 22 minutes, and 70% B was used from 22.1 to 32 minutes.

The MS analysis was performed in the Selective Ion Monitoring (SIM) mode with monitoring ions at m/z 369.3 (for cholesterol), 376.3 (for cholesterol-d7), 367.3 (for desmosterol), and 373.3 (for desmosterol-d6). The concentration in each sample was calculated based on the calibration curve of the measurement target.

### (3) Statistical Analysis

The statistical significant difference between the mean of the cholesterol level, the desmosterol level, or the ratio of desmosterol level to cholesterol level in blood plasma in patients with dementia with Lewy bodies, patients with Alzheimer's disease, or patients with frontotemporal lobar degeneration and the mean in elderly healthy subjects was determined using Tukey's multiple comparison test. The results are shown in Table 1.

### (4) Receiver Operating Characteristic Curve

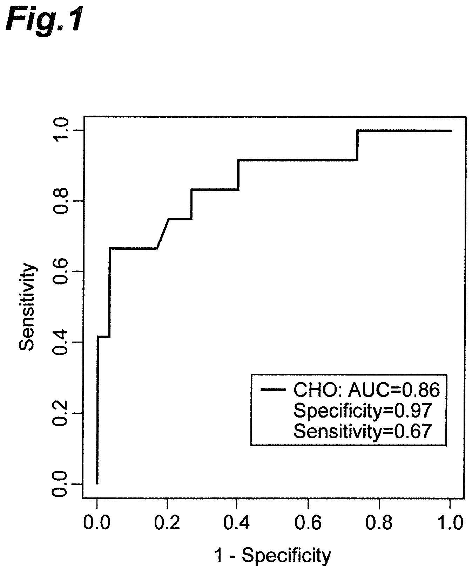

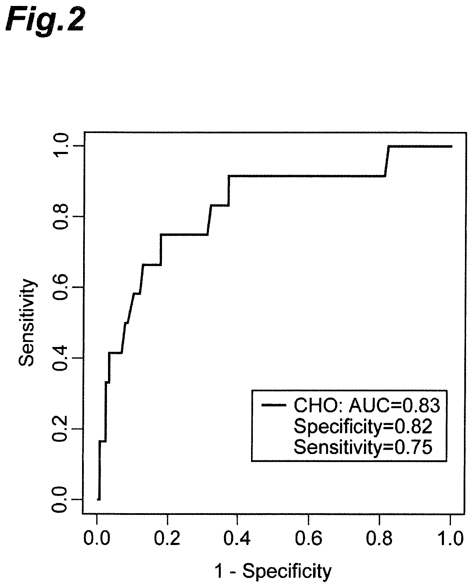

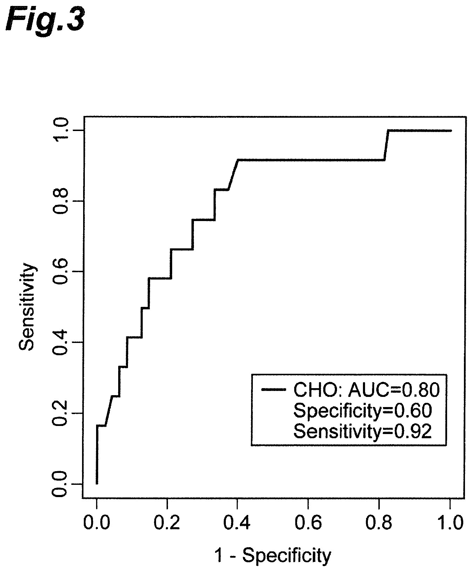

The accuracy of diagnosis using the cholesterol level, the desmosterol level, and the ratio of desmosterol level to cholesterol level in elderly healthy subjects, patients with dementia with Lewy bodies, patients with Alzheimer's disease, and patients with frontotemporal lobar degeneration was evaluated using the receiver operating characteristic curve. The receiver operating characteristic curves of the cholesterol level, the desmosterol level, and the ratio of desmosterol level to cholesterol level were generated using the receiver operating characteristic of the basic package of R. Figure 1 shows a receiver operating characteristic curve of cholesterol levels in blood plasma from elderly healthy subjects and patients with dementia with Lewy bodies. Figure 2 shows a receiver operating characteristic curve of cholesterol levels in blood plasma from patients with Alzheimer's disease and patients with dementia with Lewy bodies. Figure 3 shows also a receiver operating characteristic curve of cholesterol levels in blood plasma from patients with frontotemporal lobar degeneration and patients with dementia with Lewy bodies.

**[Table 1]**

| | DES (ng/mL) | CHO (mg/mL) | DES/CHO (ng/mg) |
|---|---|---|---|
| Elderly Healthy Subjects | 681±229 | 1**.**69±0**.**26 | 401±118 |
| AD | 410±182 ** | 1.66±0.29 n.s. | 247±973 ** |
| DLB | 434±171 ** | 1.30±0.28 ** | 336±112 n.s. |
| FTLD | 637±279 n.s. | 1.64±0.34 n.s. | 386±151 n.s. |

Data are presented as mean ± standard deviation.
**: statistically significantly different from the level in elderly healthy subjects (P = 0.01)
n.s.: not statistically significantly different from the level in elderly healthy subjects
AD: patients with Alzheimer's disease
DLB: patients with dementia with Lewy bodies
FTLD: patients with frontotemporal lobar degeneration
DES: desmosterol level
CHO: cholesterol level
DES/CHO: ratio of desmosterol level to cholesterol level

## Claims

1. A method for evaluating an effect of a candidate substance of a therapeutic agent for dementia with Lewy bodies, comprising:
measuring a desmosterol (DES) level and cholesterol (CHO) level in blood from a non-human animal before administration of the candidate substance;
measuring the DES level and CHO level in blood from the non-human animal after administration of the candidate substance; and
providing an indication that the candidate substance is likely to be effective in treatment of dementia with Lewy bodies when at least one selected from the DES level and CHO level in blood from the non-human animal after administration of the candidate substance is increased as compared with the DES level and CHO level in blood from the non-human animal before administration of the candidate substance.

2. A method for evaluating an effect of a candidate substance of a therapeutic agent for dementia with Lewy bodies, comprising:
measuring a DES level and CHO level in blood from a first non-human animal to which the candidate substance is not administered;
measuring the DES level and CHO level in blood from a second non-human animal to which the candidate substance is administered; and
providing an indication that the candidate substance is likely to be effective in treatment of dementia with Lewy bodies when at least one selected from the DES level and CHO level in blood from the second non-human animal to which the candidate substance is administered is higher than the DES level and CHO level in blood from the first non-human animal to which the candidate substance is not administered.

3. A method for evaluating an effect of a candidate substance of a therapeutic agent for dementia with Lewy bodies, comprising:
measuring a DES level and CHO level in blood from a subject before administration of the candidate substance;
measuring the DES level and CHO level in blood from the subject after administration of the candidate substance;
providing an indication that the candidate substance is likely to be effective in treatment of dementia with Lewy bodies when at least one selected from the DES level and CHO level in blood from the subject after administration of the candidate substance is increased as compared with the DES level and CHO level in blood from the subject before administration of the candidate substance.

4. A method for assisting diagnosis of dementia with Lewy bodies, comprising:
measuring a DES level and CHO level in blood from a subject and
providing an indication that the subject is likely to have dementia with Lewy bodies when at least one selected from the DES level and CHO level in blood from the subject is lower than a reference value.

5. A method for diagnosing dementia with Lewy bodies, comprising:
measuring a DES level and CHO level in blood from a subject and
determining that the subject has dementia with Lewy bodies when at least one selected from the DES level and CHO level in blood from the subject is lower than a reference value.

6. A method for selecting a patient that is likely to benefit from a therapeutic effect of a candidate substance of a therapeutic agent for dementia with Lewy bodies, comprising:
measuring a DES level and CHO level in blood from a subject and
providing an indication that the subject is a patient to be administered with the candidate substance of a therapeutic agent for dementia with Lewy bodies when at least one selected from the DES level and CHO level in blood from the subject is lower than a reference value.

7. A method for distinguishing among dementia with Lewy bodies, Alzheimer's disease, and frontotemporal lobar degeneration, comprising:
measuring a DES level and CHO level in blood from a subject and
providing an indication
(1) that the subject is likely to have dementia with Lewy bodies rather than the Alzheimer's disease and the frontotemporal lobar degeneration when the CHO level in blood from the subject is lower than a reference value; or
(2) that the subject is likely to have dementia with Lewy bodies rather than the Alzheimer's disease and the frontotemporal lobar degeneration when the DES level in blood from the subject is lower than a reference value and a ratio of the DES level to the CHO level (DES/CHO) is not different from a reference ratio.

8. A method for distinguishing among dementia with Lewy bodies, Alzheimer's disease, and frontotemporal lobar degeneration, comprising:
measuring a DES level and CHO level in blood from a subject and
providing an indication
(1) that the subject is likely to have dementia with Lewy bodies rather than Alzheimer's disease and frontotemporal lobar degeneration when the CHO level in blood from the subject is lower than a reference value;
(2) that the subject is likely to have dementia with Lewy bodies or Alzheimer's disease rather than frontotemporal lobar degeneration when the DES level in blood from the subject is lower than a reference value;
(3) that the subject is likely to have dementia with Lewy bodies rather than Alzheimer's disease and frontotemporal lobar degeneration when the DES level in blood from the subject is lower than a reference value and DES/CHO is not different from a reference value; or
(4) that the subject is likely to have Alzheimer's disease rather than dementia with Lewy bodies and frontotemporal lobar degeneration when DES/CHO in blood from the subject is lower than a reference value.
